# EUROPEAN PATENT APPLICATION

(11) **EP 3 656 488 A1**
(43) Date of publication of application: **27.05.2020**
(21) Application number: 17918160.7
(22) Date of filing: 18.07.2017
(51) Int. Cl.: B22F 1/02

(54) **FUNCTIONAL COMPOSITE PARTICLE AND PREPARATION METHOD THEREFOR**

(71) Applicant: Naxau New Materials (Zhejiang) Co., Ltd., Pinghu Economic-Technological Development Zone Jiaxing Zhejiang 314200 (CN); Aodoz Medical Technology Corporation, Zhejiang 314200 (CN)
(72) Inventor: YUAN, Ansu, Zhejiang 314200 (CN)
(74) Representative: Klunker IP Patentanwälte PartG mbB
(86) International application number: PCT/CN2017/093391
(87) International publication number: WO 2019/014854

(57) **Abstract**

The present invention relates to functional composite particles and the preparation method thereof. One embodiment of the present invention provides a functional composite particle including an inner core and a shell layer, wherein the inner core is consisted of functional metallic particles and has an outer surface, while the shell layer is a physical vapor deposition (PVD) ceramic layer consisted of biocompatible ceramic materials, and is attached to the outer surface of the inner core. The shell layer is a crystalline structure thereby allowing the ionic functional metallic particles to be sustained-released to the outside of the shell layer from the inner core via crystal boundaries. In the embodiment of the present invention, biocompatible ceramic materials are used to cover the outside surface of the functional metallic particles which have specific functions via the PVD process so as to form functional composite particles. The ionic functional metallic particles of the functional composite particles are sustained-released via crystal boundaries of the shell layer, leading to longer action time of the functional metallic particles.

## Description

### Technical field

The present invention relates to the technical field of composite materials, and in particular, to a functional composite particle and a preparation method thereof.

### Background

Biocompatible ceramics not only have the mechanical compatibility and stability of ceramics, but also have affinity and biological activity with biological tissue, and can be applied in biological, medical, chemical, and other fields related to human body or animal body. However, single biocompatible ceramics have some limitations in practical application. For example, when the biocompatible ceramics are applied in artificial joints, it is easy to cause bacterial infections and, more seriously, local complications of bones may occur.

Combining metallic particles having specific functions with biocompatible ceramics is a solution to the limitations of the biocompatible ceramics above. For example, in patent CN10293614A, a given amount of metallic silver powder is mixed in a ceramic raw material, and the mixture is sintered under a normoxic or oxygen-enhanced environment to form ceramics that can release silver ions, such that a certain antibacterial effect is achieved. However, in the sintering process of the ceramics, excessive temperature would make it difficult to locate the metallic silver at a fixed position, resulting in that the metallic silver and the ceramic material are not evenly distributed in the finished product; moreover, silver in high temperature is also easy to be oxidized by oxygen in the environment, and thus the antibacterial effect of silver ions would be lost. In addition, the bonding force between the metallic silver and the ceramic material is weak, and during specific use, the release time of silver ions is relatively short, so it is difficult to achieve a lasting antibacterial effect.

Therefore, the existing biocompatible ceramics can be further improved.

### Summary of the invention

In order to improve the property of the existing biocompatible ceramics and enhance the biological safety and specific functionality thereof, one of the purposes of the present invention is to provide a functional composite particle and a preparation method thereof.

According to an embodiment of the present invention, the functional composite particle includes an inner core and a shell layer, wherein the inner core is consisted of functional metallic particles and has an outer surface, while the shell layer is a physical vapor deposition (PVD) ceramic layer consisted of biocompatible ceramic materials, and is attached to the outer surface of the inner core. The shell layer is a crystalline structure thereby allowing the ionic functional metallic particles to be sustained-released to the outside of the shell layer from the inner core via crystal boundaries.

Another embodiment of the present invention provides a preparation method of the functional composite particle, comprising the following steps: by means of an evaporation-condensation process, putting a solid metal block composed of functional metallic particles into a crucible, and evaporating the solid metal block by heating to a vacuum PVD process furnace for condensation; and depositing a PVD ceramic layer composed of a biocompatible ceramic material on the outer surface of the functional metallic particles in the condensed state by means of a PVD process.

Another embodiment of the present invention also provides an application of the functional composite particle. The functional composite particle is used for being coated on the surface of metal, cloth, ceramic, or plastic or implanted in metal, cloth, ceramic, or plastic.

In the embodiments of the present invention, biocompatible ceramic materials are used to cover the outside surface of the functional metallic particles which have specific functions via the PVD process so as to form functional composite particles. On the one hand, this coating structure of the functional composite particle can prevent the functional metallic particles from being oxidized by ambient oxygen, and on the other hand, the ionic state of the functional metallic particles can be sustained-released through the crystal boundaries of the shell layer, such that the action time of the functional metallic particles can be prolonged.

### Brief description of the drawings

Fig. 1 shows a metallographic image of a functional composite particle according to an embodiment of the present invention;
Fig. 2 shows a metallographic image of another functional composite particle according to an embodiment of the present invention;
Fig. 3 shows an enlarged view of a portion of the metallographic image of the functional composite particle shown in Fig. 1; and
Fig. 4 shows a structure diagram of a functional composite particle according to an embodiment of the present invention.

### Detailed description of preferred embodiments

For a better understanding of the spirit of the present invention, the functional composite particle provided in embodiments of the present invention is further described in detail below with reference to the drawings and the specific embodiments. The advantages and features of the embodiments of the present invention would be clearer according to the following description and the claims.

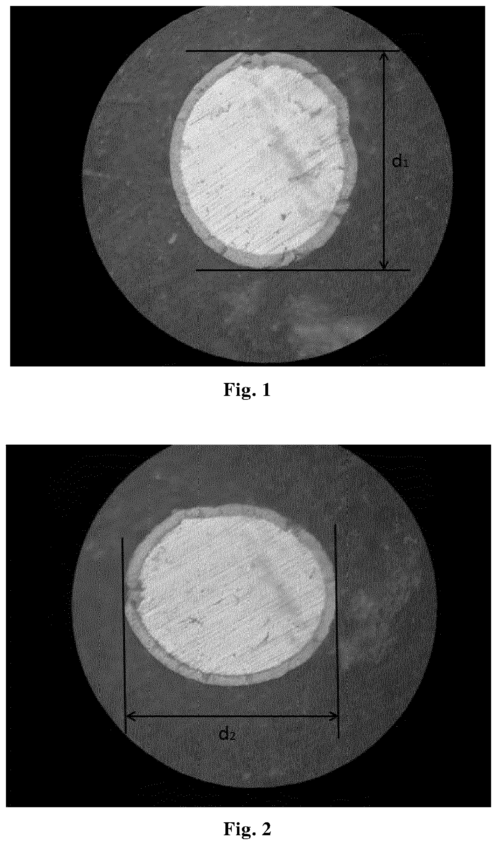

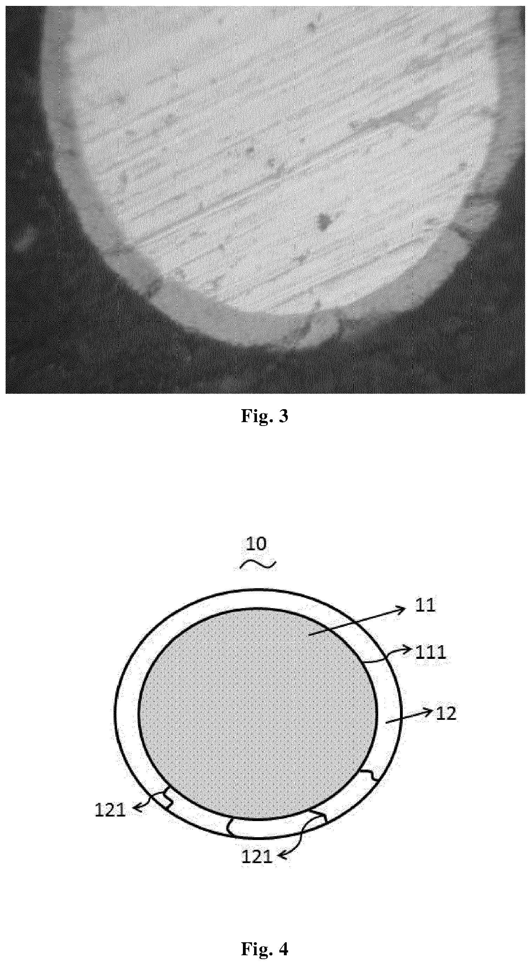

It should be noted that Figs. 1 to 3 are metallographic images captured using an electron microscope, and the diagram in Fig. 4 is in a simplified form and adopts a non-precise scale and is only used for help to conveniently and clearly describe the embodiments of the present invention.

Specifically, Fig. 1 shows a metallographic image of a functional composite particle according to an embodiment of the present invention. Fig. 2 shows a metallographic image of another functional composite particle according to an embodiment of the present invention. Fig. 3 shows an enlarged view of a portion of the metallographic image of the functional composite particle shown in Fig. 1. Fig. 4 shows a structure diagram of a functional composite particle according to an embodiment of the present invention.

With reference to Figs. 1 to 4, a functional composite particle 10 comprises an inner core 11 and a shell layer 12. The inner core 11 is composed of functional metallic particles and has an outer surface 111. The shell layer 12 is attached to the outer surface 111 of the inner core 11 and is a PVD ceramic layer consisting of a biocompatible ceramic material.

In an embodiment of the present invention, the functional metallic particles of the inner core 11 have a particle diameter of 5 nm to 5 mm; and the shell layer 12 is a biocompatible ceramic layer consisting of a metal oxide or metal nitride composed of Zr, Ti, or Al, or a mixture of the metal oxide and the metal nitride, and has a thickness of 5 nm to 50000 nm, preferably 50 nm to 5000 nm, and surface hardness of 1000 HV to 4500 HV, preferably 3000 HV to 4000 HV.

In an embodiment of the present invention, the shell layer 12 is a biocompatible ceramic layer consisting of ZrN, TiN, AlTiN, or Al₂O₃.

In an embodiment of the present invention, the functional metallic particles of the inner core 11 are antibacterial metallic particles, which are Ag metallic particles, Zn metallic particles, Cu metallic particles, or a mixture thereof.

In another embodiment of the present invention, the functional metallic particles of the inner core 11 are growth-promoting metallic particles, which are Ca metallic particles, K metallic particles, Mg metallic particles, or a mixture thereof.

As shown in Figs. 3 and 4, the shell layer 12 is of a crystalline structure and has a crystal boundary 121 which provides a passage leading to the outside of the shell layer 12 for the ionic state of the functional metallic particles in the inner core 11. During the use of the functional composite particle, since the shell layer is of a crystalline structure, the functional metallic particles in the inner core 11 are sustained-released to the outside of the shell layer 12 through the crystal boundary 121 in the form of an ionic state. According to the functional composite particle provided by the present invention, the shell layer 12 coated on the outer surface 111 of the inner core 11 can effectively prevent the functional metallic particles in the inner core 11 from contacting the ambient oxygen to avoid premature oxidation; and on the other hand, the functional metallic particles in the inner core 11 can also be sustained-released through the crystal boundary 121 of the shell layer 12, such that the action time of the functional metallic particles are prolonged.

The preparation method of the functional composite particle 10 shown in Figs. 1 to 4 comprises the following steps:
by means of an evaporation-condensation process, putting a solid metal block composed of functional metallic particles into a crucible, and evaporating the solid metal block by heating to a vacuum PVD process furnace for condensation, so as to form an inner core 11 which has an outer surface 111; and
depositing a PVD ceramic layer composed of a biocompatible ceramic material on the outer surface 111 of the functional metallic particles (the inner core 11) in the condensed state by means of a PVD process, to form a shell layer 12, wherein the shell layer 12 is of a crystalline structure, has a crystal boundary 121, and allows the ionic state of the functional metallic particles in the inner core 11 to be sustained-released to the outside of the shell layer 12 through the crystal boundary 121.

In an embodiment of the present invention, the particle diameter of the functional metallic particles after condensation is affected by the heating power of the heating source. In an embodiment of the present invention, the solid metal block composed of functional metallic particles is heated by using an electron gun as the heating source, wherein the current intensity of the electron gun is in a range of 60 A to 300 A, preferably 150 A to 250 A.

In an embodiment of the present invention, the step of forming the PVD ceramic layer by means of a PVD process comprises: introducing nitrogen or oxygen with a purity of 99.999% into the vacuum PVD process furnace; at a bias voltage of 0 V to 1000 V, opening a target containing a biocompatible ceramic material, with an arc current of 120 A to 200 A; and depositing a PVD ceramic layer on the outer surface of the functional metallic particles in the condensed state by means of the PVD process.

In the embodiments of the present invention, the shell layer 12 may be formed using a conventional PVD device by means of a conventional PVD process.

In the embodiments of the present invention, the functional composite particles have different functions depending on the kind of the functional metallic particles. For example, Ag metallic particles, Zn metallic particles, Cu metallic particles, or a mixture thereof correspond to antibacterial functional composite particles, and Ca metallic particles, K metallic particles, Mg metallic particles, or a mixture thereof correspond to growth-promoting functional composite particles.

Functional composite particles can be further coated on the surface of metal, cloth (cotton, nonwoven fabric, etc.), ceramic, or plastic. For example, the functional composite particles may be coated on the surface of a metal product, such as an orthopedic instrument, an artificial bone scaffold, or an artificial joint, to form an antibacterial coating or a growth-promoting coating, may be coated on the surface of a plastic product to form an antibacterial coating, and may also be coated on the surface of a nonwoven fabric to form an antibacterial coating, etc.

Moreover, the functional composite particles can also be implanted in metal, fabric, ceramic, or plastic to form a product having a specific function directly.

This is further illustrated in combination with some more preferred embodiments of the present invention as follows.
**Embodiment 1**
   By means of an evaporation-condensation process, putting a Ag metal block into a crucible, and evaporating the Ag metal block by heating with an electron gun at a current intensity of 200 A to a vacuum PVD process furnace for condensation; and
   introducing nitrogen with a purity of 99.999% into the vacuum PVD process furnace; at a bias voltage of 80 V to 100 V, opening a target containing Ti, with an arc current of 120 A to 200 A; and depositing a TiN ceramic layer on the outer surface of the Ag metallic particles in the condensed state by means of the PVD process, so as to form powder of TiN-Ag composite particles, the particle diameter d₁ of which is 68 nm, as shown in Fig. 1.
**Embodiment 2**
   By means of an evaporation-condensation process, putting a Cu metal block into a crucible, and evaporating the Cu metal block by heating with an electron gun at a current intensity of 200 A to a vacuum PVD process furnace for condensation; and
   introducing nitrogen with a purity of 99.999% into the vacuum PVD process furnace; at a bias voltage of 80 V to 100 V, opening a target containing Ti, with an arc current of 120 A to 200 A; and depositing a TiN ceramic layer on the outer surface of the Cu metallic particles in the condensed state by means of the PVD process, so as to form powder of TiN-Cu composite particles, the particle diameter d₂ of which is 154 nm, as shown in Fig. 2.
**Embodiment 3**
   By means of an evaporation-condensation process, putting a Zn metal block into a crucible, and evaporating the Zn metal block by heating with an electron gun at a current intensity of 200 A to a vacuum PVD process furnace for condensation; and
   introducing oxygen with a purity of 99.999% into the vacuum PVD process furnace; at a bias voltage of 80 V to 100 V, opening a target containing Al, with an arc current of 120 A to 200 A; and depositing a TiN ceramic layer on the outer surface of the Zn metallic particles in the condensed state by means of the PVD process, so as to form powder of TiN-Zn composite particles having a growth-promoting effect.
**Embodiment 4**
   By means of an evaporation-condensation process, putting a Mg metal block into a crucible, and evaporating the Mg metal block by heating with an electron gun at a current intensity of 200 A to a vacuum PVD process furnace for condensation; and
   introducing nitrogen with a purity of 99.999% into the vacuum PVD process furnace; at a bias voltage of 80 V to 100 V, opening a target containing a mixture of AlTi, with an arc current of 120 A to 200 A; and depositing an AlTiN ceramic layer on the outer surface of the Mg metallic particles in the condensed state by means of the PVD process, so as to form powder of AlTiN-Mg composite particles having a growth-promoting effect.

The functional composite particles obtained in the above embodiments 1 and 2 were respectively coated on the surfaces of plastic articles as samples of the following antibacterial effect experiments. At the same time, another identical plastic article, the surface of which was not coated with the functional composite material, was taken as a control. Test method: please refer to ISO22196:2011.

Please refer to Table I and Table II for experimental data.

**Table I**

| Experimental strains | Inoculum concentrations (cfu/mL) | Amounts of inoculum liquid (mL) | Logarithmic values of the number of bacteria obtained after elution with different contact times (cfu/cm²) | | | Values of the antimicrobial activity |
|---|---|---|---|---|---|---|
| | | | / | "0 hour" | "24 hours" | |
| *Staphylococcus aureus* ATCC6538P | 1.4×10⁵ | 0.05 | Sample | / | 1.00 | 3.8 |
| | | | Control | 3.83 | 4.85 | |
| *Escherichia coli* ATCC8739 | 1.4×10⁵ | 0.05 | Sample | / | 1.00 | 5.0 |
| | | | Control | 3.83 | 6.00 | |

**Table II**

| Experimental strains | Inoculum concentrations (cfu/mL) | Amounts of inoculum liquid (mL) | Logarithmic values of the number of bacteria obtained after elution with different contact times (cfu/cm²) | | | Values of the antimicrobial activity |
|---|---|---|---|---|---|---|
| | | | / | "0 hour" | "24 hours" | |
| *Staphylococcus aureus* ATCC6538P | 1.4×10⁵ | 0.05 | Sample | / | 1.00 | 3.8 |
| | | | Control | 3.83 | 4.85 | |
| *Escherichia coli* | 1.4×10⁵ | 0.05 | Sample | / | 5.93 | 0.1 |
| ATCC8739 | | | Control | 3.85 | 6.00 | |

| | | | | | | |
|---|---|---|---|---|---|---|
| Note: The values of the antimicrobial activity in the tables are the values of the antimicrobial activity of the samples with respect to the controls. | | | | | | |

It can be known from tables I and II that compared with the plastic which is not coated with the functional composite particles provided in embodiments 1 and 2 of the present invention, the plastic coated with the functional composite particles obviously has a higher antimicrobial property.

In the embodiments of the present invention, the biocompatible ceramic material is coated on the outer surface of functional metallic particles having a specific function by means of the PVD process to form a functional composite particle. The ionic state of the functional metallic particles in the functional composite particle can be sustained-released through the crystal boundaries of the shell layer, such that the action time of the functional metallic particles can be prolonged.

The technical content and technical features of the present invention have been described in detail, but various substitutions and modifications may be made by those skilled in the art without departing from the spirit of the invention, based on the teachings and disclosures of the present invention. Therefore, the scope of protection of the present invention should not be limited to the content disclosed by the embodiments, but rather encompasses various substitutions and modifications that do not depart from the invention and are covered by the claims of this patent application.

## Claims

1. A functional composite particle, including:
an inner core, consisted of functional metallic particles and has an outer surface; and
a shell layer, the shell layer is a physical vapor deposition (PVD) ceramic layer consisted of biocompatible ceramic materials, and is attached to the outer surface of the inner core,
wherein the shell layer is a crystalline structure thereby allowing the ionic functional metallic particles to be sustained-released to the outside of the shell layer from the inner core via crystal boundaries.

2. The functional composite particle of Claim 1, wherein the functional metallic particles are antibacterial metallic particles, the antibacterial metallic particles are Ag metallic particles, Zn metallic particles, Cu metallic particles, or a mixture thereof.

3. The functional composite particle of Claim 1, wherein the functional metallic particles are growth-promoting metallic particles, the growth-promoting metallic particles are Ca metallic particles, K metallic particles, Mg metallic particles, or a mixture thereof.

4. The functional composite particle of Claim 1, wherein the functional metallic particles have a particle diameter of 5 nm to 5 mm.

5. The functional composite particle of Claim 1, wherein the PVD ceramic layer is a biocompatible ceramic layer consisting of a metal oxide or metal nitride composed of Zr, Ti, or Al, or a mixture of the metal oxide and the metal nitride.

6. The functional composite particle of Claim 1, wherein the PVD ceramic layer is a biocompatible ceramic layer consisting of ZrN, TiN, AlTiN, or Al₂O₃.

7. The functional composite particle of Claim 1, wherein the PVD ceramic layer has a thickness of 5 nm to 50000 nm and a surface hardness of 1000 HV to 4500 HV.

8. A preparation method of functional composite particles, comprising the following steps:
by means of an evaporation-condensation process, putting a solid metal block composed of functional metallic particles into a crucible, and evaporating the solid metal block by heating to a vacuum PVD process furnace for condensation; and
depositing a PVD ceramic layer composed of a biocompatible ceramic material on the outer surface of the functional metallic particles in the condensed state by means of a PVD process.

9. The preparation method of the functional composite particle of Claim 8, wherein the functional metallic particles are antibacterial metallic particles, the antibacterial metallic particles are Ag metallic particles, Zn metallic particles, Cu metallic particles, or a mixture thereof.

10. The preparation method of the functional composite particle of Claim 8, wherein the functional metallic particles are growth-promoting metallic particles, the growth-promoting metallic particles are Ca metallic particles, K metallic particles, Mg metallic particles, or a mixture thereof.

11. The preparation method of the functional composite particle of Claim 8, wherein the PVD ceramic layer is a biocompatible ceramic layer consisting of ZrN, TiN, AlTiN, or Al₂O₃.

12. The preparation method of the functional composite particle of Claim 8, wherein the particle diameter of the functional metallic particles after condensation are changed by adjusting heating power.

13. The preparation method of the functional composite particle of Claim 8, wherein the step of forming the PVD ceramic layer by means of a PVD process comprises: introducing nitrogen or oxygen with a purity of 99.999% into the vacuum PVD process furnace; at a bias voltage of 0 V to 1000 V, opening a target containing a biocompatible ceramic material, with an arc current of 120 A to 200 A; and depositing a PVD ceramic layer on the outer surface of the functional metallic particles in the condensed state by means of the PVD process.

14. Uses of the functional composite particle of any one of Claims 1-7, being used for being coated on the surface of metal, cloth, ceramic, or plastic or implanted in metal, cloth, ceramic, or plastic.
